# EUROPEAN PATENT APPLICATION

(11) **EP 2 030 632 A1**
(43) Date of publication of application: **04.03.2009**
(21) Application number: 07115492.6
(22) Date of filing: 03.09.2007
(51) Int. Cl.: A61K 49/00, A61K 9/50, C08K 9/04, C08J 3/03

(54) **A carrier particle for use in bio-applications**

(71) Applicant: UMC Utrecht Holding B.V., 3584 CM Utrecht (NL)
(72) Inventor: The designation of the inventor has not yet been filed
(74) Representative: Hatzmann, Martin

(57) **Abstract**

The invention provides a carrier particle comprising a core of silica and/or alumina which core has a diameter in the range of from 10 nm to 10 µm, a hydrophobic layer enveloping the core, and a layer of amphiphilic molecules enveloping the hydrophobic layer. The invention further relates to processes for preparing said particle and the use of said particle. The particle suitably carries one or more diagnostic and/or one or more therapeutic agents for use in bio-applications.

## Description

The present invention relates to a carrier particle for use in bio-applications, and processes for preparing said carrier particle.

Carrier particles for use in bio-applications are, for instance, known from US 2003/0206859 A1. In said patent specification amine-terminated silica particles have been described which can be coupled with a diagnostic or therapeutic agent. Such carrier particles are generally highly charged due to the presence of amine groups, which makes them less bio-applicable. Additionally, freely exposed amine groups may react or interact with undesired species in a biological environment. Further, such particles leave relatively little room for adjusting their surface for bio-applications.

Object of the present invention is to provide a carrier particle which is more attractive for bio-applications and which shows an improved biocompatibility, whereas at the same time an improved flexibility is established for adjusting its surface for bio-applications.

It has now been found that this can be realized when use is made of carrier particles that are provided with a hydrophobic layer which is enveloped with a layer made of amphiphilic molecules.

Accordingly, the present invention relates to a carrier particle comprising a core of silica and/or alumina which core has a diameter in the range of from 10 nm to 10 µm, a hydrophobic layer enveloping the core, and a layer of amphiphilic molecules enveloping the hydrophobic layer.

The diameter of the core of silica and/or alumina to be used in accordance with the present invention is preferably in the range of from 15 nm to 1 µm, more preferably in the range of from 25 nm to 250 nm.

The hydrophobic layer of the present carrier particle can be made in different manners.

Suitably, the hydrophobic layer is formed from the reaction between hydrophilic groups that are present at the outer surface of the core and an organic compound having at least one hydrophilic end group and a hydrophobic tail, whereby a covalent bond is formed between the hydrophilic groups at the outer surface of the core and the organic compound. It will be understood that the hydrophilic groups at the surface of the core are the Si-OH and/or Al-OH end groups that are present on the surface of the core.

Preferably, the hydrophobic tail of the organic compound to be used in accordance with the present invention comprise one or more branched or unbranched alkyl, alkylene, alkylyne and/or phenyl groups which groups each contain 1-30 carbon atoms. More preferably, said one or more groups each contain 4-24 carbon atoms. Most preferably, the hydrophobic tail comprises an unbranched alkyl, alkylene, alkylyne and/or phenyl group which contains 4-24 carbon atoms.

Suitably, the hydrophilic end group is an alcohol group.

Suitable organic compounds include primary or secondary monohydric alcohols (RCOH), as described in US 2,801,185 and US 2,657,149.

Suitable straight chain alcohols to be used as the organic compound include methyl, ethyl, n-propyl, n-butyl, n-amyl, n-hexyl, n-heptyl, n-octyl, n-nonyl, n-decyl, n-undecyl, n-dodecyl, n-tetradecyl, n-hexadecyl, and n-octadecyl alcohol. Examples of branched alcohols are isopropyl alcohol, isobutyl alcohol, or 2,2,4-trimethyl-1-hexanol. Examples of suitable secondary alcohols are 2-pentanol, 2-octanol, or 2,4-dimethyl-3-pentanol. Examples of alicyclic alcohols are cyclohexanol or menthol. Suitable aromatic alcohols include benzyl alcohol, 2-phenyl-ethanol, alpha-methylbenzyl, and 3-phenyl-2-propene-1-ol. Suitable ethylenic or acetylenic unsaturated alcohols include oleyl cis-9-octadecene-1-ol or propargyl (2-propyn-1-ol) alcohol. Any combination of the above-mentioned alcohols can suitably be used in accordance with the present invention. Preferably, the organic compound is a saturated primary alcohol. It will be understood that when these primary alcohols are used in the condensation reaction with the OH-end groups that are present at the surface of the core, alkoxy groups will result.

In another embodiment of the present invention, the hydrophobic layer is formed from the reaction between hydrophilic groups that are present at the outer surface of the core and an organosilane, whereby a covalent bond is formed between the hydrophilic groups at the outer surface of the core and the organosilane.

Preferably, the organosilane has the formula R¹ₙ-Si-(OR²)₄₋ₙ, wherein R¹ is an alkyl group containing 1-30 carbon atoms, R² is an alkyl group containing 1-7 carbon atoms, and n ranges from 1-3.

Organosilanes that can suitably be used in accordance with the present invention include trialkoxysilanes having the formula RSi(OR²)₃, wherein R can be an aliphatic or aryl organic group containing 1-20 carbon atoms, such as n-butyl, n-hexyl, n-heptyl, n-octyl, t-butyl, 3-butenyl, and phenyl. R may suitably comprise functional epoxy, acrylate or methacrylate groups or hetero atoms such as N and S. R² can suitably be an alkyl group containing 1-7 carbon atoms. Suitable examples of such trialkoxysilanes include methyltrimethoxysilane, propyltriethoxysilane, octyltrimethoxysilane, dodecyltrimethoxysilane, octadecyltrimethoxysilane, octadecyltriethoxysilane, methyltriisopropoxysilane, gamma chloropropyltrimethoxysilane, 3-acryloxypropyltrimethoxysilane, 2-methacryloxyethyltrimethoxysilane, 2-acryloxyethyltrimethoxysilane, 3-methacryloxypropyltriethoxysilane, 2-methacryloxyethyltriethoxysilane, 2-acryloxyethyltriethoxysilane, gamma glycidoxypropyltrimethoxysilane, gamma methacryloxypropyltrimethoxysilane, gamma glycydoxypropyltriethoxysilane, beta glycydoxyethyltrimethoxysilane, 3,3,3-trifluoropropyltrimethoxysilane, 3,4-epoxycyclohexyl-ethyltriethoxysilane, phenyltrimethoxysilane, vinyltriethoxysilane, vinyltrimethoxyethoxysilane, vinyltris(betamethoxyethoxy)silane, n-beta(aminoethyl) gamma aminopropyltrimethoxysilane, gamma mercaptopropyltrimethoxysilane, and beta cyanoethyltriethoxysilane.

Suitable examples of dialkoxysilanes that can be used in accordance with the present invention are dimethyldimethoxysilane, dibutyldimethoxysilane, dioctyldimethoxysilane, and dioctyldiethoxysilane. Suitable examples of monoalkoxysilanes are trimethylmethoxysilane, tributylmethoxysilane, tributylethoxysilane, and trioctylmethoxysilane.

Also di-functional organosilane having the formula R^{a} R^{b} SiX₂ or a tri-functional organosilane having the formula Rₐ SiX₃ can suitably be used, wherein R^{a} and R^{b} can be the same or different and each is an aliphatic or aryl chain, fluoroalkyl, aryl, or arylmethyl, X is Cl, Br, I, or OR⁴, wherein R⁴ is a Cl -C4 alkyl. Preferred di- and tri-functional organosilanes include dialkyldichlorosilanes such as dimethyldichlorosilane and alkyltrichlorosilanes such as methyltrichlorosilane. Any combination of the above-mentioned organosilanes can suitably be used in accordance with the present invention.

Organosilanes that can be used in accordance with the present invention have in detail been described in US 4,644,077; US 2003/0035888; and US 6,159,540.

Other methods to obtain hydrophobic silica, as mentioned by R.K. Iler (The Chemistry of Silica, Wiley, 1979) can also suitably be used in accordance with the present invention. Suitable examples of such other methods include the use of organic cations (see e.g. US 2,692,863; US 3,485,592 and US 2,273,040) and the use of organic anions (see e.g. US 3,625,856), which organic cations or anions are attached to the outer surface of the silica and/or alumina core. Hence, in accordance with the present invention the hydrophobic layer can suitably be formed by attachment of organic cations or organic anions to hydrophilic groups that are present at the outer surface of the core, whereby the attachment is in the form of an ionic interaction. Other suitable examples of such methods include the use of a carboxyl group instead of an alcohol group for the condensation reaction (see e.g. M.L. Hair, Infrared Spectroscopy in Surface Chemistry, 1967, Dekker, New York), and the use of hydrophobic polymers to coat the silica and/or alumina core (see for example R. Laible, K. Hamann, Advan. Colloid Interface Sci. 13, 65 (1980)). Therefore, in accordance with the present invention the hydrophobic layer can suitably be formed by attachment of hydrophobic polymers to hydrophilic groups that are present at the outer surface of the core, whereby the attachment is in the form of a covalent bond or an ionic interaction.

Preferably, the hydrophobic layer has a thickness in the range of from 0.5 nm to 5 nm, more preferably in the range of from 1 nm to 3 nm.

In accordance with the present invention use is made of a layer of amphiphilic molecules that envelopes the hydrophobic layer. It will be appreciated that amphiphilic molecules are molecules that each comprise both a hydrophobic part and a hydrophilic part, which molecules may form micelles or bilayers in an aqueous environment.

Suitable examples of amphiphilic molecules that can be used in accordance with the present invention include lipids, block copolymers, and any mixture thereof. Suitable examples of lipids include glycerides and phospholipids preferably containing at least two hydrocarbon chains. The hydrocarbon chains may be branched and comprise side groups with for example 1 to 4 carbon atoms. The hydrocarbon chains may be saturated or unsaturated and are preferably fatty acids. Suitable fatty acids include, but are not limited to, butanoic acid, hexanoic acid, octanoic acid, decanoic acid, dodecanoic acid, tertradodecanoic acid, hexadecanoic acid, 9-hexadecenoic acid, octadecanoic acid, 12-hydroxy-9-octadecanoic acid, 11-octadecenoic acid, 9,12-octadecadienoic acid, 9,12,15-octadecatrienoic acid, 9,12,15-octadecatrienoic acid, 9,12,15-octadecatrienoic acid, 9-eicosenoic acid, 5,8,11,14-eicosatetraenoic acid, 5,8,11,14,17-eicosapentaenoic acid, docosanoic acid, 13-docosenoic acid, 4,7,10,13,16,19-docosahexaenoic acid and tetracosanoic acid.

Block co-polymers are copolymers in which a hydrophobic monomer is linked to a hydrophilic monomer. Any block copolymer can be used which forms a polymeric micelle or bilayer with a hydrophobic inside and a hydrophilic outside in an aqueous environment. The amphiphilic layer formed by block copolymer moieties can contain two, three or more blocks (types of monomers). The total number of moieties in the block copolymer can be up to 2000

Suitable examples of hydrophobic monomers include styrene, glycolide, lactide, butadiene, vinylbutylether, ethylenepropylene, ethylethylene, isobutylene, methylacrylic acid, propyl methacrylic acid, butyl methacrylic acid, pentyl acrylic acid, cyclopentadienylmethylnorbornene, N-butylacrylic acid, caprolactone, propyleneglycol, siloxane, phosphazene and azoline. Suitable examples of hydrophilic monomers include methacrylic acid, acrylic acid, maleic acid, maleimides, vinyl pyridine, vinyl imidazole, vinyl acetic acid, n-alkylacrylamides, n,n-dialkylacrylamides, n,n-propylmethacrylamide, n-isopropylmethacrylamide, trialkylaminoethylmethacrylamide, trimethylammoniumethylmethacrylamide, n-alkylvinylpyridimium, sulfonic acid, styrene sulfonic acid, hydroxyethylmethacrylamide, methyletherdiethylene glycolmethacrylamide, acrylonitriles, ethylene imine, oxazoline, ethyleneoxide, propyleneoxide, vinylpyrrolidone, ethyleneglycol, ethylene glycolacrylic acid, ethylene glycolmethacrylamide, amino acids, and monosacharides such as glucose, galactose, mannose, and fructose.

Preferably, the amphiphilic molecule is a lipid. Preferably, the lipid is a phospholipid. The phospholipid may be any ester of glycerol and phosphoric acid that is further linked by ester bonds to two fatty acids.

Suitable examples of lipids also include paramagnetic lipids, fluorescent lipids, radiolabeled lipids, charged lipids, and functional lipids and lipids comprising polyethylene glycol (PEGylated lipids). In respect of PEGylated it is observed that these lipids display an improved circulation half life and that they can attractively be used for functionalizing the carrier particle. In the layer of amphiphilic molecules use can be made of a mixture of two or more different types of amphiphilic molecules. The layer of amphiphilic molecules establishes that the carrier particle is water soluble and that it is highly bio-applicable.

Suitably, the layer of amphiphilic molecules has a thickness in the range of from 1 nm to 50 nm, preferably a thickness in the range of from 3 nm to 25 nm.

The carrier particle in accordance with the present invention comprises a core of silica and/or alumina. This means that the core may consist solely of silica or alumina, or it may comprise a mixture of silica and alumina. Preferably, the core comprises silica. The silica to be used may be amorphous, crystalline, hydrated, solvated, or dry, and may exist in a variety of particulate and aggregation forms and may be porous or non-porous.

The core particles may be synthesized by any method known in the art. A common method for the preparation of colloidal silica particles is based on the base-catalyzed hydrolysis and condensation of alkoxysilanes. Suitable examples include, but are not limited to, the sol-gel method (see e.g. W. Stöber, A. Fink, and E. Bohn, J. Colloid Interface Sci., 26, 62 (1968)), and the reverse microemulsion method (see e.g. Osseo-Asare, K., and Arriagada, F. J., Colloids Surf. 50, 321 (1990)). Suitable examples for the preparation of colloidal alumina include but are not limited to the sol-gel method using alumina alkoxides (see e.g. B.E. Yoldas, Am. Cer. Soc. Bull., 54,289 (1975) and US 3,357,791), and the reverse microemulsion method (see e.g. E. Ponthieu, E Payen, and J. Grimblot, J. Non-Cryst. Solids,147, 598 (1992)). Suitable examples for making silica/alumina particles include, but are not limited to, the methods as for instance described in US 3,007,878, US 3,252,917, and US 3,956,171:

The carrier particle according to the present invention suitably comprises in addition one or more diagnostic agents and/or one or more therapeutic agents. The diagnostic and/or therapeutic agents may be present in the core, in the hydrophobic layer or in the layer of amphiphilic molecules, or they may be attached to the outer surface of the core, the outer surface of the hydrophobic layer or the outer surface of the layer of the amphiphilic molecules.

A suitable example of a method for incorporating diagnostic agents in silica particles is for instance well described in WO 01/88540 A1.

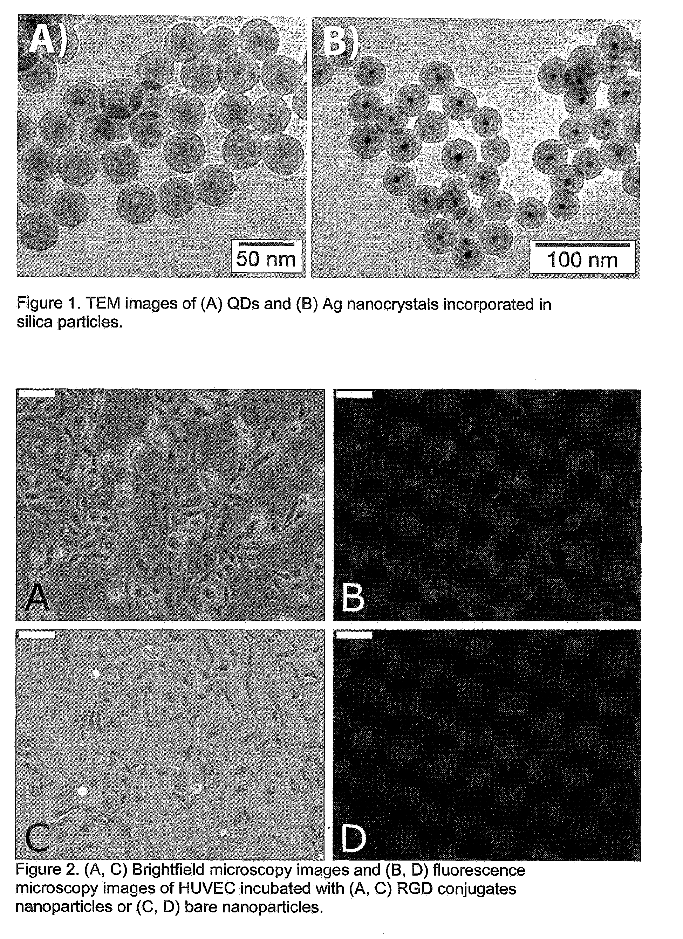

Suitable diagnostic agents include, for instance, contrast agents for a variety of imaging techniques, such as: semiconductor nanoparticles, fluorescent dyes, phosphor nanoparticles, rare-earth metal complexes, metal nanoparticles, magnetic nanoparticles, radio-active components or any combination of these contrast agents. These contrast agents can be applied for fluorescence imaging, magnetic resonance imaging (MRI), positron emission tomography (PET), single photon emission computed tomography (SPECT), electron microscopy (EM), ultrasound (US), computed tomography (CT), photon tomography and magnetic particle imaging. The semiconductor, magnetic, or metal nanoparticles that are incorporated in the core of the carrier particle can have a diameter in the range between 1 nm to 500 nm in size.Preferably, the semiconductor nanoparticles have a diameter between 2 nm and 10 nm. The magnetic nanoparticles have preferably a diameter between 5 nm and 50 nm, and the diameter of the metal nanoparticles preferably ranges between 10 nm and 100 nm. One or more fluorescent semiconductor nanoparticles (quantum dots, QDs) can be incorporated for fluorescence imaging, for example CdSe QDs (see Figure 1A). Fluorescent dyes, phosphor nanoparticles or rare-earth metal complexes can be used for this purpose as well. One or more magnetic nanoparticles can be incorporated in the core of the carrier particle in order to introduce contrast for MRI detection, for example Fe₂O₃ particles. Inclusion of molecules with paramagnetic ions in the hydrophobic layer or the layer of amphiphilic molecules can also suitably be used for this purpose, for example Gd³⁺ in a chelate complex like gadolinium diethylenetriaminepentaacetic acid-bis stearylamide (Gd-DTPA-BSA). One or more metal nanoparticles can be used as a contrast agent for computed tomography or electron microscopy, for example gold or silver-containing nanoparticles (see Figure 1B). Radio-active components like positron emitters can also suitably be used for PET imaging and radio-active gamma ray emitters can be used for SPECT imaging. Any combination of the contrast agents as mentioned hereinabove can be used per carrier particle, to obtain a multi-modality contrast agent for *in vitro* or *in vivo* imaging. A general advantage of the encapsulation of these contrast agents in silica particles is the reduced (cyto)toxicity of the resulting carrier particle, making it very attractive for bio-applications. In addition, the use of the carrier particle offers a large flexibility for combining different contrast agents within one single particle.

The therapeutic agents to be used in accordance with the present invention include drugs, bioactive molecules, metal nanoparticles, magnetic nanoparticles, DNA, mRNA, microRNA and silencing RNA.

The present invention also relates to the use of a suspension according to the present invention for the treatment and/or prevention of a disease.

Suitable examples of diseases include atherosclerosis, cancer, arthritis, diabetes, myocardial infarction, neurological disorders such as inflammation after stroke, multiple sclerosis and Alzheimer's disease.

In addition, the present invention also relates to a diagnostic composition comprising a carrier particle according to the present invention which comprises one or more diagnostic agents, a powder according to the present invention which comprises a number of carrier particles according to the present invention which comprise one or more diagnostic agents, or a diagnostic suspension according to the present invention.

In accordance with the present invention the carrier particle may comprise one or more diagnostic agents and one or more therapeutic agents.

In addition, the present invention also relates to a therapeutic composition consisting of a carrier particle according to the present invention which comprises one or more therapeutic agents, a powder which comprises a number of carrier particles according to the present invention, or a therapeutic suspension of said particles.

In accordance with the present invention the carrier particle can be functionalized by attaching linking molecules that can bind to one or more targeting ligands. The chemical linker can be attached to the hydrophobic part or the hydrophilic part of the amphiphilic layer. Preferably, the linker molecule is attached to the hydrophilic part and is exposed on the surface. The chemical linker comprises a moiety that can couple to biological moieties. Examples of such moieties include, but are not limited to, amine, thiol, maleimide, biotin, succinimidyl, carboxyl, aldehyde and/or nitriloacetic groups. The targeting ligands can be any molecule with affinity for a specific target, receptor, or cell type. Suitable examples of such targeting ligands include peptides, proteins, antibodies, antibody fragments, small molecules, sugars, DNA and RNA and other types of targeting molecules. The targeting ligands can be attached to the chemical linker by any method known in the art.

Targeting of hydrophobic or poorly water soluble drugs, e.g. chlorines for photodynamic therapy or cytostatic agents like doxorubicin and cisplatin, can, for instance, be realised by their inclusion in the hydrophobic layer. Targeting of hydrophilic drugs, e.g. antibodies, peptides, small molecules, etc, can be established by attaching such drugs to, for instance, the lipids and PEG-lipids. In another embodiment of the present invention, cationic or anionic amphiphiles can be used to modify the charge of the outer surface of the carrier particle to establish nucleotide/agent delivery or transfection.

The present invention also relates to a set of carrier particles according to the present invention. Such a set of carrier particles can suitably be a powder. Hence, the present invention also relates to a powder comprising a number of carrier particles according to the present invention.

Further, the present invention also relates to a suspension comprising a carrier particle or a powder according to the present invention.

Such a suspension can suitably be a therapeutic and/or a diagnostic suspension.

The suspension according to the present invention can also suitably be a Magnetic Resonance Imaging (MRI), X-ray Computed Tomography (CT), Positron Emission Tomography (PET), Single Photon Emission Computed Tomography (SPECT), fluorescence imaging, magnetic particle imaging suspension. The present invention also relates to the use of the suspension according to the present invention in one of the imaging techniques as mentioned hereinabove.

The present invention also relates to a process for preparing carrier particles according to the present invention, which process comprises the steps of:
(a) providing particles which comprises a core of silica and/or alumina which core has a diameter in the range of from 10 nm to 10 µm, and a hydrophobic layer enveloping the core;
(b) dispersing the particles in the presence of amphiphilic molecules in a hydrophobic solvent;
(c) evaporating at least part of the solvent;
(d) adding water to the product obtained in step (c); and
(e) recovering the carrier particles.

In addition, the present invention relates to a process for preparing carrier particles according to the present invention, which process comprises the steps of:
(a) providing particles which comprises a core of silica and/or alumina which core has a diameter in the range of from 10 nm to 10 µm, and a hydrophobic layer enveloping the core;
(b) forming an emulsion of the particles with amphiphilic molecules, a hydrophobic solvent and water;
(c) evaporating at least part of the hydrophobic solvent; and
(d) recovering the carrier particles.

Suitably, in the latter process, the emulsion is formed by (a) dispersing the particles in the presence of the amphiphilic molecules in the hydrophobic solvent, after which water is added to the dispersion so obtained or the dispersion so obtained is added to water, or the emulsion is formed by (b) dispersing the particles in the hydrophobic solvent, after which the dispersion so obtained is added to a mixture comprising water and the amphiphilic molecules or the mixture comprising water and the amphiphilic molecules is added to the dispersion so obtained.

The particles to be provided in step (a) of the processes as described hereinabove are suitably obtained by adding to particles of silica and/or alumina which have a diameter in the range of from 10 nm to 10 µm an organic compound having at least one hydrophilic end group and a hydrophobic tail under conditions which allow hydrophilic groups that are present at the outer surface of the core to react with the organic compound, whereby a covalent bond is formed between the hydrophilic groups at the surface of the core and the organic compound.

The organic compound can be any of the organic compounds which have been defined hereinabove.

In another embodiment of the present invention, the particles provided in step (a) are obtained by adding to particles of silica and/or alumina which have a diameter in the range of from 10 nm to 10 µm an organosilane under conditions which allow hydrophilic groups that are present at the outer surface of the core to react with the organosilane, whereby a covalent bond is formed between the hydrophilic groups at the surface of the core and the organosilane.

The organosilane to be used for this purpose can be any of the organosilanes as defined hereinbefore.

The hydrophobic solvent to be used in accordance with the present invention can suitably be selected from, but is not limited to, the group consisting of chloroform, dichloormethane, ethers (like diethyl ether), esters (like ethyl acetate), hydrocarbons (like hexane, heptane, octane, nonane, decane, dodecane and cyclohexane), and aromatic solvents (like toluene and benzene). In other examples, a mixture of one of the hydrophobic solvents mentioned with a hydrophilic solvent like methanol, ethanol, can be used. Preferably, the hydrophobic solvent is chloroform.

### Example

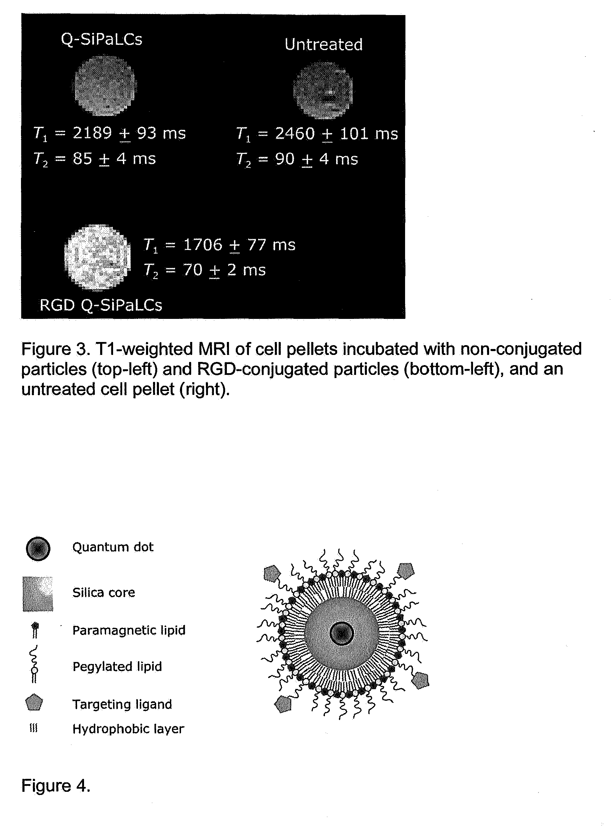

To obtain bio-applicable silica carrier particles in accordance with the present invention, silica particles having a diameter of 43 nm were functionalized with a contrast agent comprising a core/shell CdSe/CdS/CdZnS/CdZnS quantum dot (QD) of 7 nm which was incorporated in the centre of the silica particles (for example Figure 1a). The functionalized particles were then immersed in an excess of octadecanol and the mixture obtained was heated to a temperature of 170°C for 3 hours to form covalent Si-O-C bonds on the outer surface of the silica particles through a condensation reaction yielding hydrophobic silica particles.
In a second step the hydrophobic silica particles were enclosed in a lipidic layer. The lipids used were a combination of 50% Gd-DTPA-BSA based, 40% pegylated (PEG-DSPE), and 10% pegylated lipids functionalized with maleimide. The lipids were dispersed in chloroform together with the hydrophobic silica particles. The mixture so obtained was then added drop wise to water at a temperature of 90°C under vigorous stirring. In this way, a clear dispersion of hydrophobic silica particles enclosed in a lipidic monolayer in water was obtained.
The bio-applicability and target specific uptake of the particles so obtained was demonstrated by fluorescence imaging and MRI of human umbilical vein endothelial cells (HUVEC) that were incubated with either non-targeted particles or with αvβ3-integrin targeted particles. To that end multiple RGD-peptides, specific for αvβ3-integrin receptors that are expressed by proliferating HUVEC, were subsequently conjugated to the multiple maleimide groups that were exposed by the functionalized pegylated lipids. The specificity of the targeted QD/silica/lipid particles for HUVEC was investigated and compared to untargeted particles (see Figure 2). As can be seen from the bright fluorescence of the QDs, the QD/silica/lipid particles were specifically taken up by the HUVEC, while the non-specific uptake for not-functionalized particles was negligible. Due to the inclusion of Gd-DTPA-BSA based lipids the particles had a high relaxivity, which made them also detectable with MRI. A T1 weighted image of the different cell pellets is depicted in Figure 3 and clearly demonstrates a higher signal intensity for the cell pellet of cells incubated with the RGD conjugated particles as compared to control cell pellets. A schematic drawing of the carrier particles obtained in this Example is shown in Figure 4.

## Claims

1. A carrier particle comprising a core of silica and/or alumina which core has a diameter in the range of from 10 nm to 10 µm, a hydrophobic layer enveloping the core, and a layer of amphiphilic molecules enveloping the hydrophobic layer.

2. A particle according to claim 1, wherein the core has a diameter in the range of from 25 nm to 250 nm.

3. A particle according to claim 1 or 2, wherein the hydrophobic layer is formed from the reaction between hydrophilic groups that are present at the outer surface of the core and an organic compound having at least one hydrophilic end group and one or more hydrophobic tails, whereby a covalent bond is formed between the hydrophilic groups at the outer surface of the core and the organic compound.

4. A particle according to claim 3, wherein the hydrophobic tail comprises one or more alkyl, alkylene, alkylyne and/or phenyl groups which groups each contain 1-30 carbon atoms.

5. A particle according to claim 4, wherein the hydrophobic tail comprises one or more alkyl, alkylene, alkylyne and/or phenyl groups which groups each contain 4-24 carbon atoms.

6. A particle according to any one of claims 3-5, wherein the hydrophilic end group of the organic compound is an alcohol.

7. A particle according to claim 1 or 2, wherein the hydrophobic layer is formed from the reaction between hydrophilic groups that are present at the outer surface of the core and an organosilane, whereby a covalent bond is formed between the hydrophilic groups at the outer surface of the core and the organosilane.

8. A particle according to 7, wherein the organosilane has the formula R¹ₙ-Si-(OR²)₄₋ₙ, wherein R¹ is an alkyl, an alkylene or a phenyl group containing 1-30 carbon atoms, R² is an alkyl group containing 1-7 carbon atoms, and n ranges of from 1-3.

9. A particle according to claims 1 or 2, wherein the hydrophobic layer is formed by attachment of organic cations or organic anions to hydrophilic groups that are present at the outer surface of the core, whereby the attachment is in the form of an ionic interaction.

10. A particle according to claims 1 or 2, wherein the hydrophobic layer is formed by attachment of hydrophobic polymers to hydrophilic groups that are present at the outer surface of the core, whereby the attachment is in the form of a covalent bond or an ionic interaction.

11. A particle according to any one of claims 1-10, wherein the hydrophobic layer has a thickness in the range of from 0.5 nm to 5 nm.

12. A particle according to any one of claims 1-11 wherein the amphiphilic molecules are lipids or block copolymers.

13. A particle according to claim 12, wherein the amphiphilic molecules are lipids.

14. A particle according to claim 13, wherein the lipids are phospolipids.

15. A particle according to any one of claims 1-14, wherein the layer of amphiphilic molecules has a thickness in the range of from 1 nm to 50 nm.

16. A particle according to any one of claims 1-15, wherein the core comprises silica.

17. A particle according to any one of claims 1-16, comprising in addition one or more diagnostic agents and/or one or more therapeutic agents.

18. A particle according to claim 17, wherein one or more diagnostic agents are present in the core, linked to the outer surface of the core, attached to outer surface of the hydrophobic layer present in the hydrophobic layer, present in the amphiphilic layer, or attached to the amphiphilic layer.

19. A particle according to claim 17 or 18, wherein the one or more therapeutic agents are present in the core, the hydrophobic layer or amphiphilic layer, or attached to the amphiphilic layer.

20. A particle according to any one of claims 1-19, wherein one or more targeting ligands are attached to the outer surface of the amphiphilic layer.

21. A powder comprising a number of particles as described in any one of claims 1-20.

22. A suspension comprising one or more particles as described in any one of claims 1-20.

23. A suspension according to claim 22, wherein said suspension is a therapeutic suspension.

24. A suspension according to claim 22, wherein said suspension is a Magnetic Resonance Imaging (MRI), X-ray Computed Tomography (CT), Positron Emission Tomography (PET), Single Photon Emission Computed Tomography (SPECT), photon tomography, fluorescence imaging or magnetic particle imaging suspension.

25. Use of a suspension according to claim 22 in one of the imaging techniques as mentioned in claim 24.

26. Use of a suspension according to claim 23 for the treatment and/or prevention of a disease.

27. A diagnostic composition comprising a particle according to any one of claims 17-20, a powder according to claim 21 or a suspension of claim 24.

28. A therapeutic composition comprising a particle according to any one of claims 17-20, a powder according to claim 21 or a suspension of claim 23.

29. A process for preparing carrier particles according to claim 1 or 2, comprising the steps of:
(a) providing particles which comprise a core of silica and/or alumina which core has a diameter in the range of from 10 nm to 10 µm, and a hydrophobic layer enveloping the core;
(b) dispersing the particles in the presence of amphiphilic molecules in a hydrophobic solvent;
(c) evaporating at least part of the solvent;
(d) adding water to the product obtained in step (c); and
(e) recovering the carrier particles.

30. A process for preparing carrier particles according claim 1 or 2, comprising the steps of:
(a) providing particles which comprise a core of silica and/or alumina which core has a diameter in the range of from 10 nm to 10 µm, and a hydrophobic layer enveloping the core;
(b) forming an emulsion of the particles with amphiphilic molecules, a hydrophobic solvent and water;
(c) evaporating at least part of the hydrophobic solvent; and
(d) recovering the carrier particles.

31. A process according to claim 30, wherein the emulsion is formed by (a) dispersing the particles in the presence of the amphiphilic molecules in the hydrophobic solvent, after which water is added to the dispersion so obtained or the dispersion so obtained is added to water, or the emulsion is formed by (b) dispersing the particles in the hydrophobic solvent, after which the dispersion so obtained is added to a mixture comprising water and the amphiphilic molecules or the mixture comprising water and the amphiphilic molecules is added to the dispersion so obtained

32. A process according to any one of claims 29-31, wherein the particles provided in step (a) are obtained by adding to particles of silica and/or alumina which a diameter in the range of from 10 nm to 10 µm an organic compound having at least one hydrophilic end group and a hydrophobic tail under conditions which allow hydrophilic groups that are present at the outer surface of the core to react with the organic compound, whereby a covalent bond is formed between the hydrophilic groups at the surface of the core and the organic compound.

33. A process according to any one of claims 29-31, wherein the particles provided in step (a) are obtained by adding to particles of silica and/or alumina which have a diameter in the range of from 10 nm to 10 µm an organosilane under conditions which allow hydrophilic groups that are present at the outer surface of the core to react with the organosilane, whereby a covalent bond is formed between the hydrophilic groups at the surface of the core and the organosilane.
